Europäisches Patentamt

European Patent Office

Office européen des brevets

(19)

(11) Numéro de publication : **0 309 342 B1**

(12) **FASCICULE DE BREVET EUROPEEN**

(45) Date de publication du fascicule du brevet :
**15.01.92 Bulletin 92/03**

(51) Int. Cl.$^5$ : **A61K 35/78**

(21) Numéro de dépôt : **88402377.1**

(22) Date de dépôt : **21.09.88**

(54) **Utilisation du desmodium pour le traitement des hépatites et médicament correspondant.**

Le dossier contient des informations
techniques présentées postérieurement au
dépôt de la demande et ne figurant pas dans le
présent fascicule.

(30) Priorité : **24.09.87 FR 8713348**

(43) Date de publication de la demande :
**29.03.89 Bulletin 89/13**

(45) Mention de la délivrance du brevet :
**15.01.92 Bulletin 92/03**

(84) Etats contractants désignés :
**BE DE ES FR GB IT**

(56) Documents cités :
**Néant**

(73) Titulaire : **Tubery, Pierre**
**Lamasquère**
**F-31600 Muret (FR)**
Titulaire : **Tubery née Claustres, Anne-Marie**
**Lamasquère**
**F-31600 Muret (FR)**

(72) Inventeur : **Tubery, Pierre**
**Lamasquère**
**F-31600 Muret (FR)**
Inventeur : **Tubery née Claustres, Anne-Marie**
**Lamasquère**
**F-31600 Muret (FR)**

(74) Mandataire : **Barre, Philippe**
**Cabinet Barre-Gatti-Laforgue 95 rue des**
**Amidonniers**
**F-31069 Toulouse Cédex (FR)**

EP 0 309 342 B1

## Description

La présente invention concerne l'utilisation d'une substance pour l'obtention d'un médicament, ainsi que le médicament obtenu, en vue du traitement des hépatites virales et hépatites toxiques.

Le médicament conforme à l'invention pour le traitement des hépatites est caractérisé en ce qu'il comprend comme principe actif du Desmodium d'une espèce contenant des alcaloïdes de nature indolique. En particulier, parmi les plantes de ce genre, on choisit préférentiellement du Desmodium ascendens (parfois orthographié adscendens).

L'invention s'étend à l'utilisation de cette substance pour l'obtention d'un médicament, se présentant en particulier sous forme de plante sèche pour réaliser une décoction ou de gélules de poudre de plante sèche, en vue du traitement des hépatites.

Le Desmodium ascendens est une plante herbacée dont l'aire géographique est la zone équatoriale et les régions humides de la zone tropicale de l'Afrique. Elle fait partie des papillonacées. Elle est décrite dans la "Flore illustrée du Sénégal" (1976, Vol. V, p. 193-194). Elle est utilisée comme fourrage pour les chèvres et le bétail.

Les études pharmacologiques ont démontré qu'elle possédait une action thérapeutique dans l'hépatite virale. Les atteintes toxiques de la cellule hépatique, qu'elles soient accidentelles ou médicamenteuses (chimiothérapie anticancéreuse en particulier), ont également fait l'objet de l'étude et, quoique la statistique ne soit pas aussi homogène dans ce cas, il paraît démontré que cette substance possède également une action thérapeutique. (Dans tous les cas, cette action a été mise en évidence par la normalisation des transaminases).

Le protocole d'utilisation consiste en particulier à faire bouillir 10 à 15 grammes (selon le poids corporel de l'adulte) de la plante sèche (tiges et feuilles) dans un litre et demi d'eau pendant quinze minutes. Après filtrage, la décoction sera absorbée dans le courant de la journée. Le traitement devra durer de cinq (s'il a été instauré dès les deux ou trois premiers jours d'ictère, et si la forme ne présente pas d'emblée des éléments de gravité) jusqu'à trente jours et plus si la forme est plus grave ou si le traitement est plus tardif. On peut arrêter le traitement dès que les signes cliniques et biologiques sont sensiblement normaux. Le traitement peut également être effectué en broyant les plantes séchées et en conditionnant la poudre obtenue sous la forme de gélules, chaque gélule correspondant à une dose contenant de l'ordre de 200 à 300 milligrammes de poudre.

Les résultats portant sur 34 cas d'hépatites virales, qu'elles soient "A", "B" ou "non A", "non B" sont les suivants :

Dans les 11 cas où le traitement a été appliqué avant le troisième jour de l'ictère, le retour à la normale de la couleur des téguments et des urines s'est effectué après 5 jours de traitement.

Après ces 5 jours, les transaminases G.O.T. et G.P.T., qui étaient en moyenne respectivement à 730 et 640 unités, ont baissé à 65 et 58 (pour une norme de 30 et 25).

Le temps de normalisation parfaite n'a pas dépassé 20 jours dans ces 11 cas.

Dans les 14 cas où le traitement a été appliqué entre le troisième et le vingtième jour après l'apparition de l'ictère, les transaminases ont baissé au-dessous du tiers du leur valeur entre le jour 1 et le jour 10 du traitement.

A titre d'exemple : observation succinte d'un cas d'hépatite virale ayant présenté le taux le plus fort de transaminases :

M.B. né en 1966

Hépatite B diagnostiquée par son médecin traitant le 4 février 1986. Asthénie, ictère. Adressé à un service de gastroentérologie de C.H.U. qui l'adresse à Paris en raison du taux très élevé de transaminases pour l'éventualité d'une cure d'Interferon. Cette éventualité n'est pas retenue, en raison de l'amélioration spontanée des facteurs de l'hémostase, et le malade est renvoyé à Toulouse.

Le Desmodium est administré dès le 15 février 1986, l'ictère a disparu et l'état général et digestif est parfait — Bon état en décembre 1986.

Le tableau ci-dessous résume les étapes biologiques :

| Dates | 5.02.86 | 15.02.86 | 17.02.86 | 24.02.86 | 19.03.86 |
|---|---|---|---|---|---|
| Transaminases | | Début | | | |
| G.O.T. | 5 650 | de la | 90 | 62 | 25 |
| G.P.T. | 8 460 | cure | 1 500 | 140 | 31 |
| | | de | | | |
| Taux de | | Desmodium | | | |
| prothrombine | 19 % | | 100 % | | |

Enfin, dans les neuf cas où ce traitement a été instauré et dont les paramètres cliniques et biologiques étaient encore perturbés après le 20ème jour d'ictère, les résultats ont été variables :

Il y a dans les 9 cas un infléchissement des transaminases, mais la normalisation totale n'a été obtenue que dans 4 cas, après des délais de 2 à 4 mois. Les cinq autres n'ont pas été normalisés, ce fait signifiant le passage à l'hépatite chronique auto-immune.

Des hépatites d'origine toxique ont reçu la même thérapeutique : il s'agissait parfois d'un toxique absorbé volontairement par des toxicomanes ou, plus souvent, des hépatites induites par les médicaments anticancéreux (Methotrexate ou Adriamycine ou cis-dichloro-dimethyl platinium).

Dans la mesure où le personne ne présentait pas des séquelles d'une atteinte hépatique plus ancienne, les transaminases sont redevenues normales dans les 10 jours.

Ce fait a suggéré l'utilisation du Desmodium ascendens dans la prévention des effets hépatoxiques de la chimiothérapie anticancéreuse, la posologie devenant alors : 7 à 10 grammes de plante sèche à infuser dans un litre d'eau.

Cette infusion doit être absorbée tous les jours de deux jours avant à cinq jours après la chimiothérapie.

**Revendications**

**Revendications pour les Etats contractants suivants : BE, DE, FR, GB, IT**

1. Médicament pour le traitement des hépatites, comprenant comme principe actif un Desmodium d'une espèce contenant des alcaloïdes de nature indolique.

2. Médicament selon la revendication 1, constitué par du Desmodium ascendens.

3. Utilisation d'un Desmodium d'une espèce contenant des alcaloïdes de nature indolique pour l'obtention d'un médicament destiné au traitement des hépatites.

4. Utilisation selon la revendication 3 du Desmodium ascendens.

5. Utilisation selon l'une des revendications 3 ou 4 pour l'obtention d'un médicament sous la forme de plante sèche, en vue de réaliser une décoction.

6. Utilisation selon l'une des revendications 3 ou 4 pour l'obtention d'un médicament sous la forme de gélules de poudre de plante sèche.

**Revendications pour l'Etat contractant suivant : ES**

1. Utilisation d'un Desmodium d'une espèce contenant des alcaloïdes de nature indolique pour l'obtention d'un médicament destiné au traitement des hépatites.

2. Utilisation selon la revendication 1 du Desmodium ascendens.

3. Utilisation selon l'une des revendications 1 ou 2 pour l'obtention d'un médicament sous la forme de plante sèche, en vue de réaliser une décoction.

4. Utilisation selon l'une des revendications 1 ou 2 pour l'obtention d'un médicament sous la forme de gélules de poudre de plante sèche.

**Patentansprüche**

**Patentansprüche für folgende Vertragsstaaten : BE, DE, FR, GB, IT**

1. Arzneimittel für die Behandlung von Hepatitis, das als Wirkstoff ein Desmodium einer indolartige Alkaloide enthaltenden Art umfaßt.

2. Arzneimittel nach Anspruch 1, aus Desmodium ascendens bestehend.

3. Verwendung eines Desmodiums indolartige Alkaloide enthaltender Art zwecks Erzielung eines für die Behandlung von Hepatitis bestimmten Arzneimittels.

4. Verwendung des Desmodium ascendens nach Anspruch 3.

5. Verwendung nach einem der Ansprüche 3 oder 4 zwecks Erzielung eines Arzneimittels in der Form der trockenen Pflanze für die Herstellung eines Aufgusses.

6. Verwendung nach einem der Ansprüche 3 oder 4 zwecks Erzielung eines Arzneimittels in der Form von Pulver der trockenen Pflanze enthaltenden Gelatinekapseln.

**Patentansprüche für folgenden Vertragsstaat : ES**

1. Verwendung eines Desmodiums indolartige Alkaloid enthaltender Art zwecks Erzielung eines für die Behandlung von Hepatitis bestimmten Arzneimittels.

2. Verwendung des Desmodium ascendens nach Anspruch 1.

3. Verwendung nach einem der Ansprüche 1 oder 2 zwecks Erzielung eines Arzneimittels in der Form der trockenen Pflanze für die Herstellung eines Aufgusses.

4. Verwendung nach einem der Ansprüche 1 oder 2 zwecks Erzielung eines Arzneimittels in der Form von Pulver der trockenen Pflanze enthaltenden Gelatinekapseln.

**Claims**

**Claims for the following Contracting States : BE, DE, FR, GB, IT**

1. Medicament for the treatment of hepatitis, comprising by way of active substance a desmodium of a kind containing alkaloids of indolic type.

2. Medicament according to claim 1, constituted by desmodium ascendens.

3. Use of a desmodium of a kind containing alkaloids of indolic type with a view to obtaining a medicament intended for the treatment of hepatitis.

4. Use according to claim 3 of the desmodium ascendens.

5. Use according to one of claims 3 or 4 with a view to obtaining a medicament in the form of dry plant with the aim of producing a decoction.

6. Use according to one of claims 3 or 4 with a view to obtaining a medicament in the form of gelatinous capsules of the powder of the dry plant.

**Claims for the following Contracting State : ES**

1. Use of a desmodium of a kind containing alkaloids of indolic type with a view to obtaining a medicament intended for the treatment of hepatitis.

2. Use according to claim 1 of the desmodium ascendens.

3. Use according to one of claims 1 or 2 with a view to obtaining a medicament in the form of dry plant with the aim of producing a decoction.

4. Use according to one of claims 1 or 2 with a view to obtaining a medicament in the form of gelatinous capsules of the powder of the dry plant.